# EUROPEAN PATENT APPLICATION

(11) **EP 4 253 564 A1**
(43) Date of publication of application: **04.10.2023**
(21) Application number: 21898546.3
(22) Date of filing: 23.11.2021
(51) Int. Cl.: C12Q 1/686

(54) **TARGET NUCLEIC ACID AMPLIFICATION METHOD WITH HIGH SPECIFICITY AND TARGET NUCLEIC ACID AMPLIFYING COMPOSITION USING SAME**

(30) Priority: 24.11.2020 KR 20200158791
(71) Applicant: Panagene Inc., Daejeon 34027 (KR)
(72) Inventor: PARK, Jaejin, Seoul 01883 (KR); NAM, Hyo Joo, Suwon-si Gyeonggi-do 16334 (KR); KIM, Minchul, Daejeon 34007 (KR); LEE, Young, Daejeon 34140 (KR)
(74) Representative: Mewburn Ellis LLP
(86) International application number: PCT/KR2021/017237
(87) International publication number: WO 2022/114720

(57) **Abstract**

The present invention relates to a target nucleic acid amplification method capable of amplifying a very low concentration of a target nucleic acid at high specificity and a target nucleic acid amplification composition using same and, more specifically, to a method for producing an amplicon of a target nucleic acid at high specificity through a guide probe binding to a target nucleic acid if present, and a partial primer capable of binding to the guide probe and amplifying the target nucleic acid, and a polymerase chain reaction (PCR) composition for implementing the method. The target nucleic acid amplification method according to the present invention is able to amplify even a target nucleic acid present at a very low concentration because the guide probe is utilized to bind to all hybridizable nucleic acid present in a sample while helping the partial primer bind to a detection site of the target nucleic acid. In addition, having the advantage that a target nucleic acid can be detected with high specificity due to the sequence specificity of the partial primer which leads to a difference in amplification rates for nucleic acids other than the target nucleic acid, the method is useful in molecular diagnosis, prenatal diagnosis, early diagnosis, cancer diagnosis, heredity-related diagnosis, genetic trait diagnosis, diagnosis of infectious bacteria, discrimination of drug-resistant bacteria, forensic medicine, and classification of organism species.

## Description

### [Technical Field]

The present invention relates to a method for amplifying a target nucleic acid capable of amplifying a very low concentration of a target nucleic acid at high specificity and a composition for amplifying a target nucleic acid using the same, and more particularly, to a method of generating an amplicon of a target nucleic acid at high specificity using a guide probe bound to a target nucleic acid and a partial primer capable of binding to the guide probe to amplify the target nucleic acid when the target nucleic acid is present, and a composition for polymerase chain reaction (PCR) used to implement the method.

### [Background Art]

The genetic information of all living organisms on earth is the source of the unique properties of each living organism and includes bases of adenine (A), guanine (G), cytosine (C), thymine (T) and uracil (U) recorded in the order in a substance called "nucleic acid" (DNA or RNA). Therefore, revealing or identifying the sequence of these bases (nucleotide sequences) may be a process for identifying the properties of living organisms and understanding inherent metabolic mechanisms thereof.

Recently, molecular diagnostics for determining the existence or properties of living organisms by detecting or identifying a specific nucleotide sequence of living organisms has been widely used. Representative examples of pharmaceutically used molecular diagnostics include tests for detecting genetic mutations related to human cancer, detecting pathogens that cause infectious diseases that may occur in humans, and detecting harmful microorganisms present in other foods.

Most of various techniques of molecular diagnostics for specifically identifying a certain nucleotide sequence use a polymerase chain reaction (PCR) using a nucleic acid polymerase (DNA polymerase). The polymerase chain reaction is performed using a composition containing a pair of primers that can be specifically hybridized with a target nucleic acid including a specific nucleotide sequence region and a thermo-stable nucleic acid polymerase capable of causing a polymerase chain reaction using the target nucleic acid as a template based on the primers as a trigger, and a device called a "thermal cycler" that can apply a predetermined temperature to the composition in a stepwise and repeated manner. In addition, molecular diagnosis through polymerase chain reaction utilizes a nucleic acid-binding dye or probe to detect in real time a certain nucleotide sequence in a mass-produced (amplified) target nucleic acid, which is called "real-time polymerase chain reaction (real-time PCR)".

High sensitivity and high specificity are required to detect a gene mutation associated with human cancer in molecular diagnosis. The reason being that, since the mutation to be detected corresponds to a somatic mutation, false-positives may be generated if target mutations are not accurately recognized in point mutations that are admixed in very small amounts with unmutated (wild-type) normal DNA and have no significant difference from nucleotide sequence of normal gene, or in insertion/deletion mutations and gene fusion mutations that have complicated nucleotide sequence mutations.

In other words, the major requirements for tumor-specific mutation detection tests are (1) sensitivity to detect mutant DNA present at a low proportion in normal DNA and (2) specificity to minimize the false-positive errors causing normal DNA to be mis-determined as mutant DNA.

Various test methods for detecting tumor-specific mutations have been developed and used and representative methods include direct sequencing, allele-specific PCR (AS-PCR), restriction fragment length polymorphism (RFLP), TaqMan probe method, and ARMS (amplification refractory mutation system) PCR. However, these methods did not exhibit satisfactory sensitivity and specificity. Direct sequencing has the highest specificity and thus a low false positive rate, but has a disadvantage in that it is capable of detecting only when more than 20 to 30% of mutant DNA is present. Meanwhile, AS-PCR, RFLP, and TaqMan probe methods have high sensitivity but still have the problem of false positives due to low specificity.

Recently, sequencing analysis with high specificity has been gradually emphasized. For example, methods with greatly improved sensitivity and specificity such as PNA (peptide nucleic acid)-mediated PCR clamping (Sun, X., et al., 2002. Nat Biotechnol, 20: 186-189), LNA (locked nucleic acid)-mediated PCR clamping (Dominguez, P. L., et al. al., 2005. Oncogene, 24: 6830-6834), COLD-PCR (co-amplification at lower denaturation temperature PCR) (Li, J., et al., 2008. Nat. Med, 14: 579-584), and the like have been developed.

In an attempt to improve specificity, which is the drawback of AS-PCR, ARMS-PCR was developed. ARMS-PCR is based on the principle that PCR is performed only when the primers have a perfect complementary sequence to the sequence of the template, like AS-PCR. (Newton, C. R., et al., 1989. Nucl Acids Res, 17; 2503-2516). In order to determine the optimal primer with excellent specificity, disadvantageously, experimental trial and error is inevitable (Drenkard, E., et al. 2000. Plant Physiol. 124: 1483-1492).

Accordingly, the present inventors have made diligent efforts to develop a method that can remarkably increase the specificity of amplification of target nucleic acids present at a very low concentration. As a result, the present inventors found that, when PCR is performed by producing an amplification product using a guide probe capable of hybridizing with a site other than the target nucleic acid detection site, a partial primer including a sequence binding to a site other than the target nucleic acid hybridization site of the guide probe, and a sequence binding to the target nucleic acid detection site, and a specific primer that pairs with the partial primer to amplify the target nucleic acid, a very low concentration of the target nucleic acid can be detected at high specificity, and completed the present invention based thereon.

The information disclosed in this Background section is provided only for better understanding of the background of the present invention, and therefore it may not include information that forms the prior art that is already obvious to those skilled in the art.

### [Disclosure]

Therefore, it is one object of the present invention to provide a method for amplifying a very low concentration of target nucleic acid at high specificity.

It is another object of the present invention to provide a polymerase chain reaction (PCR) composition for amplifying a target nucleic acid capable of amplifying a very low concentration of target nucleic acid at high specificity.

In accordance with one aspect of the present invention, the above and other objects can be accomplished by the provision of a method of amplifying a target nucleic acid including (a) mixing a nucleic acid isolated from a sample with i) a guide probe capable of hybridizing with a site other than a detection site of the target nucleic acid,
ii) a partial primer including a sequence binding to a site other than a target nucleic acid hybridization site of the guide probe and a sequence binding to the detection site of the target nucleic acid, and
iii) a specific primer capable of pairing with the partial primer to amplify the target nucleic acid, and performing polymerase chain reaction (PCR), and
(b) determining whether or not an amplification product is present.

In accordance with another aspect of the present invention, provided is a PCR composition for amplifying a target nucleic acid including i) a guide probe capable of hybridizing with a site other than a detection site of the target nucleic acid, ii) a partial primer including a sequence binding to a site other than a target nucleic acid hybridization site of the guide probe and a sequence binding to the target nucleic acid detection site, and iii) a specific primer that pairs with the partial primer to amplify the target nucleic acid.

### [Description of Drawings]

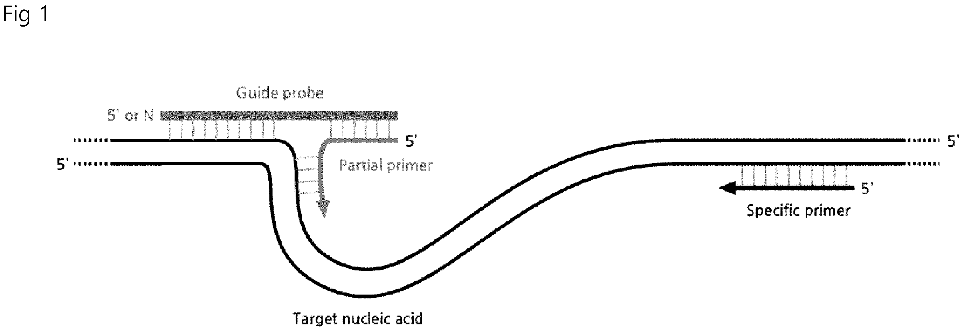

FIG. 1 illustrates hybridization relationships between a guide probe, a partial primer, a specific primer, and components necessary for one embodiment of the present invention.

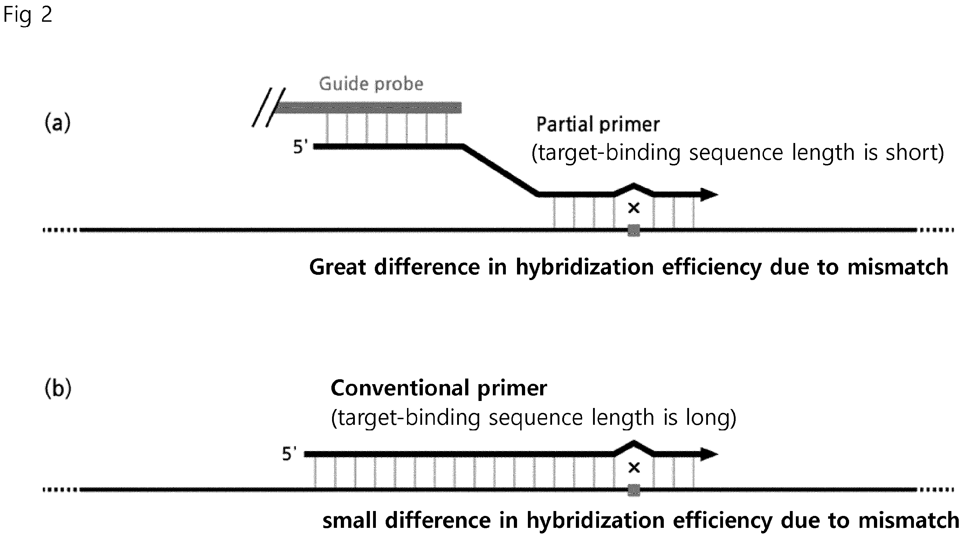

FIG. 2 shows the principle for maximizing the difference in amplification efficiency due to the difference in nucleotide sequence between target nucleic acid and non-target nucleic acid according to the present invention, more specifically, (a) shows that the difference in hybridization efficiency attributable to nucleotide sequence mismatch is large due to the small length of the part of the partial primer of the present invention that hybridizes with the target nucleic acid, resulting in maximization of the difference in amplification efficiency between normal DNA and mutant DNA and (b) shows that the difference in hybridization efficiency attributable to nucleotide sequence mismatch is small due to the great length of the part of the conventional primer that hybridizes with the target nucleic acid, thus making it difficult to distinguish between normal DNA and mutant DNA.

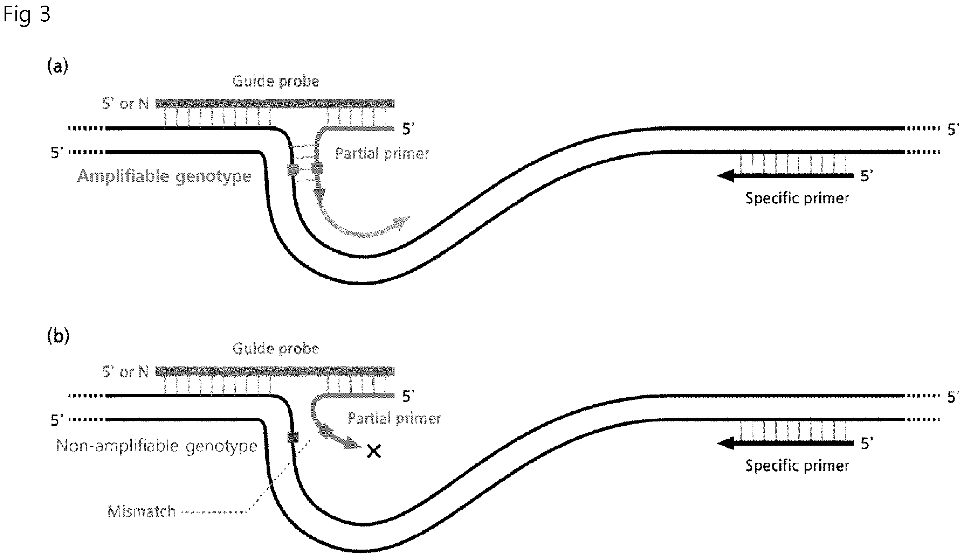

FIG. 3 shows a process of deriving the difference in amplification efficiency based on the difference in nucleotide sequence between target nucleic acid and non-target nucleic acid according to the present invention, more specifically, (a) shows that, in the presence of the target nucleic acid, with the help of a guide probe that hybridizes to a specific site of the target nucleic acid, when the partial primer that hybridizes with a part of the guide probe approaches the target amplification site of the target nucleic acid, the part of the 3' end of the partial primer is complementary to the nucleotide sequence of the target amplification site of the target nucleic acid, resulting in hybridization and extension amplification, wherein the hybridization between the guide probe and the target nucleic acid and hybridization between the guide probe and the partial primer occur earlier due to high Tm, and hybridization between the partial primer and the target nucleic acid occurs later due to low Tm resulting from the small complementary sequence length, and (b) shows that, in the presence of a non-target nucleic acid having a partial nucleotide sequence different from the target nucleic acid, even if the guide probe hybridizes with a specific site of the non-target nucleic acid, and the partial primer that hybridizes with a part of the guide probe approaches the non-target nucleic acid, hybridization and extension amplification are inhibited due to low complementarity between a part of the 3' end of the partial primer and the nucleotide sequence of the non-target nucleic acid.

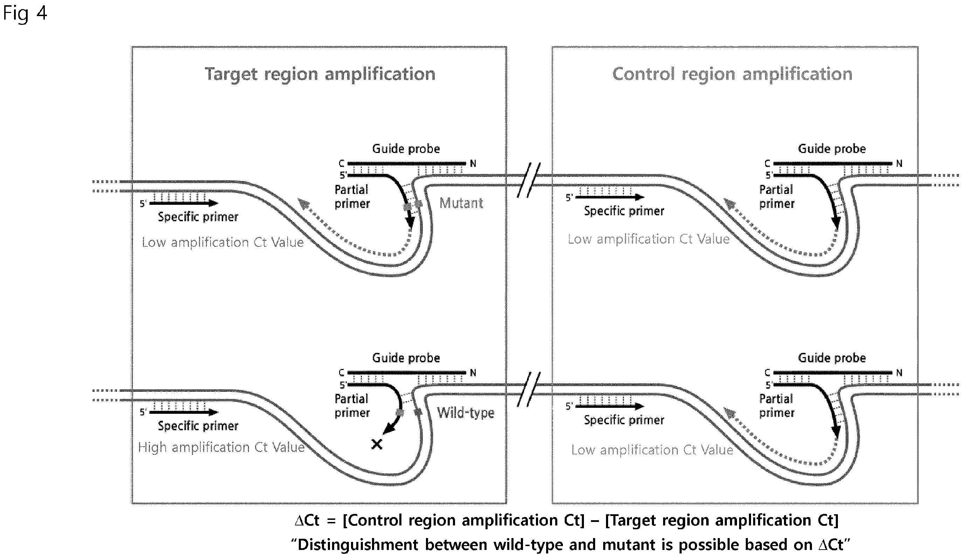

FIG. 4 is a conceptual diagram illustrating a method of detecting a specific mutation separately from a wild type according to an embodiment of the present invention.

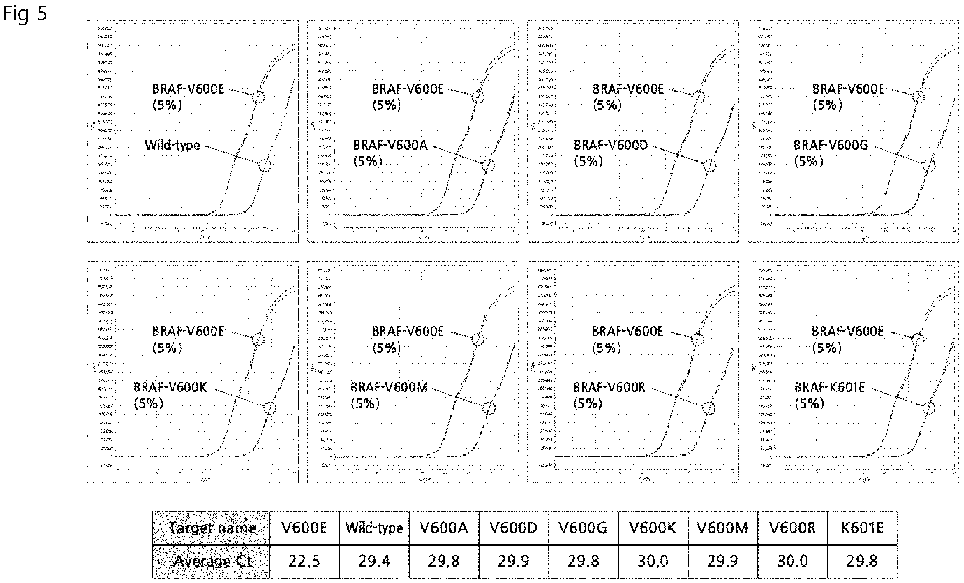

FIG. 5 shows the results of detection of the V600E mutation of the BRAF gene in the human genome with high specificity according to an embodiment of the present invention, wherein, when a partial primer having a 3' end part designed to hybridize with the genotype of the V600E mutation is used, high amplification efficiency is obtained in the presence of the V600E mutation, resulting in relatively low Ct values, whereas when wild-type and non-target mutations (V600A, V600D, V600G, V600K, V600M, V600R, K601E) are present, low amplification efficiency is obtained, resulting in higher Ct values, and as a result, the presence or absence of the V600E mutation can be determined by comparison between the Ct values.

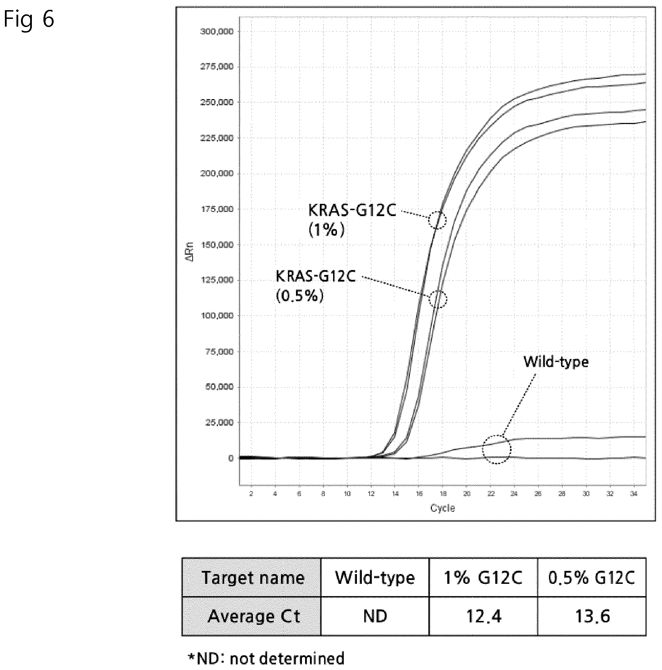

FIG. 6 shows the results of detection of the G12C mutation of the KRAS gene in the human genome with high sensitivity according to an embodiment of the present invention, wherein, when a partial primer having a 3' end part designed to hybridize with the genotype of the G12C mutation is used, high amplification efficiency is obtained in the presence of very small amounts (1% and 0.5%) of the G12C mutation, resulting in a lower Ct value, whereas, when only the wild type is present, no amplification curve is formed, and as a result, whether or not the G12C mutation is present can be determined by comparison between Ct values.

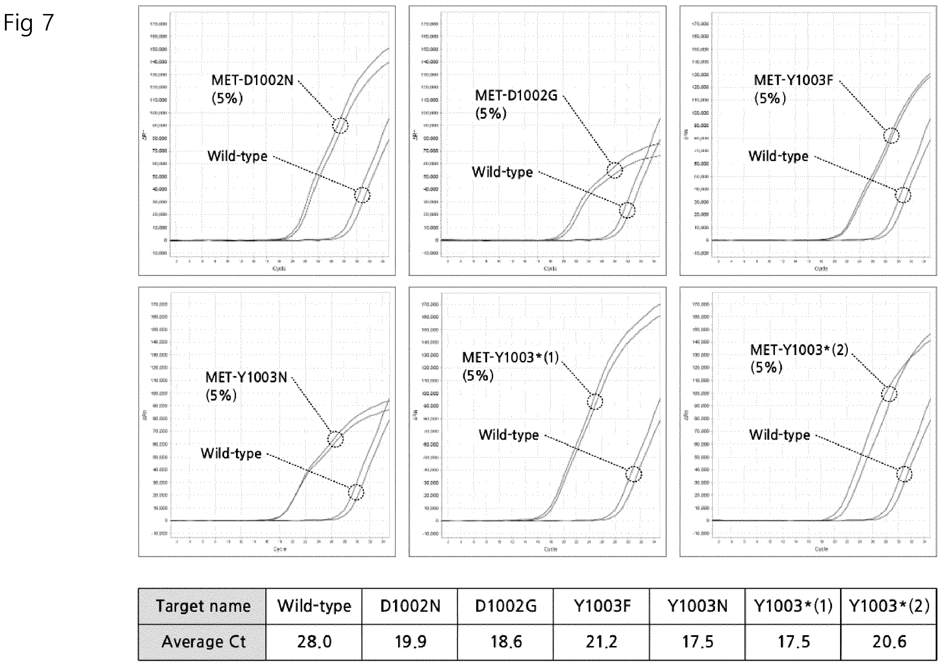

FIG. 7 shows the results of detection of D1002N, D1002G, Y1003F, Y1003N, Y1003*(1), and Y1003*(2) mutations of the MET gene in the human genome in a single reaction solution according to an embodiment of the present invention, wherein, when 6 kinds of partial primers are separately prepared and mixed such that a part of the 3' end hybridizes with the D1002N, D1002G, Y1003F, Y1003N, Y1003*(1), and Y1003*(2) mutant genotypes, high amplification efficiencies are obtained in the presence of target mutations, resulting in a lower Ct value, whereas, when only the wild type is present, low amplification efficiency is obtained, resulting in higher Ct value, and as a result, whether or not the MET mutation is present can be determined by comparison between Ct values.

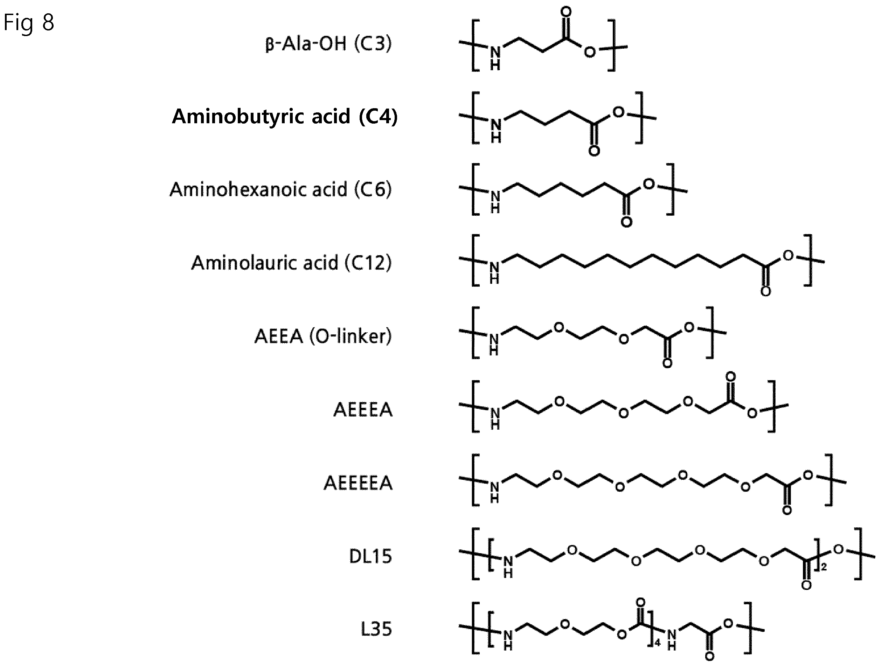

FIG. 8 shows an example of a linker that may be included in the guide probe of the present invention.

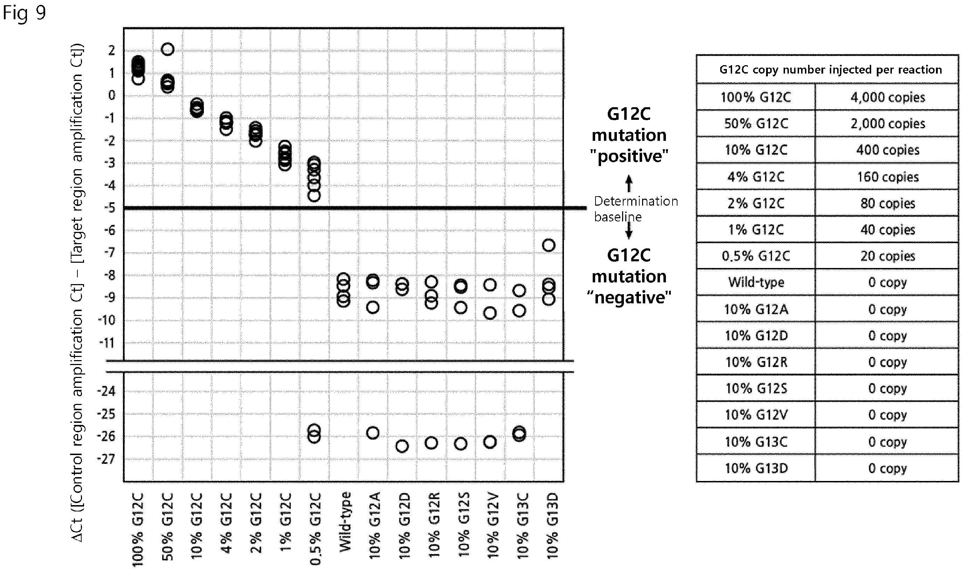

FIG. 9 shows the result of measurement of the detection limit for detecting the G12C mutation of the KRAS gene in the human genome according to one embodiment of the present invention, which indicates that, even if only about 0.5% (20 copies) of the target mutant nucleic acid per reaction is present, very high sensitivity that can be determined and excellent specificity are obtained.

### [Best Mode]

Unless defined otherwise, all technical and scientific terms used herein have the same meanings as appreciated by those skilled in the field to which the present invention pertains. In general, the nomenclature used herein is well-known in the art and is ordinarily used.

In the present invention, whether or not a very low concentration of target nucleic acid can be detected using a guide probe and a partial primer is determined.

That is, in an embodiment of the present invention, in order to specifically amplify and detect the V600E mutation, which is a GTG>GAG nucleotide sequence mutation located at exon 15, codon 600 of the human BRAF gene, a guide probe capable of specifically hybridizing with a site near codon 600 of the BRAF gene, a partial primer having a 3' end part capable of specifically hybridizing with codon 600 of the BRAF gene or a neighboring area thereof, and a specific primer capable of hybridizing with a part of the exon 15 nucleotide sequence of the BRAF gene so as to pair with the partial primer to amplify the target nucleic acid are designed. At this time, a partial nucleotide sequence at the C terminus of the guide probe is hybridized with a partial nucleotide sequence at the 5' end of the partial primer.

PCR was performed using the guide probe, partial primers and specific primers to determine whether or not the V600E can be detected in the presence of wild-type nucleic acid and various mutations present at the V600 position.

As a result, although an increase in fluorescence signal on the amplification curve was observed under all target nucleic acid injection conditions, the increase in fluorescence signal on the amplification curve was faster and a lower Ct was obtained under the condition of combination of the V600E mutant target nucleic acid, compared to under other conditions (FIG. 5).

In one aspect, the present invention is directed to a method of amplifying a target nucleic acid including (a) mixing a nucleic acid isolated from a sample with i) a guide probe capable of hybridizing with a site other than the detection site of the target nucleic acid,
ii) a partial primer including a sequence binding to a site other than a target nucleic acid hybridization site of the guide probe and a sequence binding to the target nucleic acid detection site, and
iii) a specific primer capable of pairing with the partial primer to amplify the target nucleic acid, and performing polymerase chain reaction (PCR), and
(b) determining whether or not an amplification product is present.

As used herein, the term "target nucleic acid" refers to any nucleic acid including the sequence of interest to be amplified or detected. The target nucleic acid includes gene sequences derived from several species, subspecies, or variants, or gene mutations within the same species, and include, but are not limited to, all types of DNA, including genomic DNA, mitochondrial DNA, and viral DNA, and all types of RNA including mRNA, ribosomal RNA, non-coding RNA, tRNA, viral RNA, and the like. The target nucleic acid can hybridize with the guide probe, the partial primer (partial 3' end), and the specific primer under conditions for polymerase chain reaction.

As used herein, the term "hybridization" refers to a process in which double-stranded nucleic acids are formed through hydrogen bonds between complementary single-stranded nucleic acids and has a similar meaning to the term "annealing". In a slightly broader sense, hybridization may occur either in perfect match in which complementarity between bases of two single nucleic acid strands is perfect, or even in the presence of some mismatching nucleotide sequences.

As used herein, the term "guide probe" refers to a probe that can hybridize with both a target nucleic acid and a partial primer and helps the 3' end of the partial primer to hybridize with the target nucleotide sequence of the target nucleic acid. The guide probe may be produced from any substance that is capable of hybridizing with the target nucleic acid, for example, DNA, RNA, LNA (locked nucleic acid), PNA (peptide nucleic acid), or a mixture of two or more thereof.

As used herein, the "partial primer" is characterized in that it extends through a nucleic acid polymerase by which a part of the 5' end thereof is hybridized with the guide probe and a part of the 3' end thereof is hybridized with the target nucleotide sequence of the target nucleic acid.

In a preferred embodiment of the present invention, the target nucleic acid, guide probe, and partial primer are hybridized with each other (target nucleic acid - guide probe, guide probe - partial primer, partial primer - target nucleic acid). The guide probe binds to the target nucleotide sequence of the target nucleic acid and the hybridized partial primer is disposed near the target nucleotide sequence. As a result, the 3' end of the partial primer can be more easily hybridized with the target nucleotide sequence and synthetic extension through a nucleic acid polymerase and target site amplification of the target nucleic acid are initiated.

The hybridization between the target nucleic acid and the guide probe and the hybridization between the guide probe and the partial primer occur earlier because the complementary parts therebetween are longer than the complementary part between the partial primer and the target nucleic acid and thus have a high Tm, and hybridization between the partial primer and the target nucleic acid occurs later due to the low Tm thereof.

As used herein, the term "specific primer" refers to a primer that hybridizes with a target nucleic acid without the aid of the guide probe and causes synthetic extension through a nucleic acid polymerase.

In the present invention, a site of the guide probe that hybridizes with the target nucleic acid is present at a 5' end or N-terminus and a site of the guide probe that hybridizes with the partial primer is present at a 3' end or C-terminus.

In the present invention, the guide probe may further include a linker between the site of the guide probe that hybridizes with the target nucleic acid and the site of the guide probe that hybridizes with the partial primer.

In the present invention, any compound may be used as the linker without limitation as long as it is present between the site of the guide probe that hybridizes with the target nucleic acid and the site of the guide probe that hybridizes with the partial primer to connect the two sites and does not contain a nucleic acid base, and preferably includes at least one selected from the group consisting of beta-alanine (β-Ala-OH, C3), aminobutyric acid (C4), aminohexanoic acid (C6), aminolauric acid (C12), acetoacetoxyethyl acrylate (AAEA (O-linker)), aminoethoxyethoxyethoxyacetic acid (2-[2-[2-[2-(amino) ethoxy]ethoxy]ethoxy] acetic acid, AEEEA), AEEEEA, DL15 and L35, but is not limited thereto.

In the present invention, the guide probe, partial primer, and specific primer may include oligonucleotide, LNA (locked nucleic acid), PNA (peptide nucleic acid), or a mixture thereof.

In the present invention, any nucleic acid may be used as the guide probe without limitation as long as it is capable of hybridizing with the target nucleic acid and the partial primer and is preferably PNA having a nucleotide sequence length of 10 to 500, more preferably having a nucleotide sequence length of 20 to 150.

In the present invention, the guide probe may be hybridized with an opposite strand to one strand of the target nucleic acid with which the specific primer hybridizes.

In the present invention, the partial primer further includes a spacer as a single strand oligonucleotide with a nucleotide sequence length of 1 to 100 between the sequence binding to a site other than the site of the guide probe hybridizing with the target nucleic acid and the sequence binding to the target nucleic acid detection site.

In the present invention, the spacer refers to a sequence excluding the sequence included in the partial primer hybridizing with the guide probe and the sequence included in the partial primer hybridizing with the target nucleic acid, and if necessary, may function to perform detection with the same probe even if the nucleotide sequence of the target nucleic acid is changed using a probe capable of binding to the spacer.

In the present invention, the spacer and the linker of the guide probe may function to control the hybridization efficiency of the partial primer located near the target site of the target nucleic acid based on the guide probe with the target site. This means that, when the guide probe hybridizes with the target nucleic acid at a position slightly distant from the target site, increasing the length of the spacer and linker can help the partial primer hybridize with the target site.

In the present invention, the sequence of the partial primer binding to the target nucleic acid detection site may be a sequence of 3 to 15 nucleotides.

In the present invention, the length of the amplification product may be 50 bp to 1 kbp.

In the present invention, the step (b) of determining whether or not the amplification product is present may be performed using a nucleic acid-binding dye or probe.

In the present invention, any intercalating substance or DNA minor groove-binding substance may be used as the nucleic acid-binding dye without limitation and is preferably selected from the group consisting of ethidium bromide, SYBRⓡ Green I, SYBRⓡ Gold, EvaGreen, YO-PRO-1, SYTO, BEBO and BEXTO.

In the present invention, the probe capable of binding to the amplification product may be selected from the group consisting of oligonucleotides, LNAs, PNAs, and mixtures thereof.

PNA (peptide nucleic acid) is a DNA analogue in which nucleic acid bases are linked to a peptide backbone rather than a sugar-phosphate backbone and was first synthesized by Nielsen *et al.* in 1991. PNA is an artificially synthesized gene-recognizing substance such as LNA (locked nucleic acid) or MNA (morpholino nucleic acid) and includes polyamide as the basic skeleton.

PNA has excellent affinity and selectivity, and is not degraded by existing restriction enzymes due to high stability against nucleases. In addition, PNA is advantageous in easy long-term storage due to high thermal and chemical stability thereof.

PNA forms duplexes through hybridization with a natural nucleic acid having a complementary nucleotide sequence. PNA/DNA duplexes are more stable than DNA/DNA duplexes, and PNA/RNA duplexes are more stable than DNA/RNA duplexes when they are the same length. In addition, PNA has a higher ability to detect single nucleotide polymorphisms (SNPs) than natural nucleic acids because double strands are highly unstable due to single base mismatches.

In other words, the PNA-DNA binding force is very superior to the DNA-DNA binding force and even one nucleotide mismatch usually causes a change in melting temperature (Tm) of about 15 to 20°C. The difference in binding force can be used to detect nucleotide sequence changes such as SNP (single-nucleotide polymorphism) and In/Del (insertion/deletion).

In the present invention, the probe capable of binding to the amplification product may have a nucleotide sequence partially or entirely complementary to any nucleotide sequence and target nucleic acid nucleotide sequence in the amplification product and both ends thereof are preferably connected to a reporter and a quencher.

In the present invention, signal generation of the probe is suppressed when the distance between the reporter and the quencher is short, and the signal intensity increases as the distance between the reporter and the quencher increases. In general, when the probe hybridizes with a complementary nucleotide sequence, the distance between the reporter and the quencher becomes the greatest, so that a specific nucleotide sequence can be detected through signal generation or increase in signal intensity.

In the present invention, the reporter is at least one fluorescent material selected from the group consisting of fluorescein, fluorescein chlorotriazinyl, rhodamine green, rhodamine red, tetramethylrhodamine, FITC, Oregon green, Alexa Fluor, FAM, JOE, ROX, HEX, Texas Red, TET, TRITC, TAMRA, cyanine dyes and thiadicarbocyanine dyes.

In the present invention, the quencher includes at least one selected from the group consisting of Dabcyl, TAMRA, Eclipse, DDQ, QSY, Blackberry Quencher, Black Hole Quencher, Qxl, Iowa Black FQ, Iowa Black RQ, and IRDye QC-1.

In a preferred embodiment of the present invention, the detection of the amplification product through the nucleic acid polymerase is performed through real-time PCR, and at this time, the Ct (cycle threshold) is measured by obtaining an amplification curve according to the increase of the amplification product, but is not limited thereto. The method using the Ct is based on the principle that, as the amplification product is generated and increased earlier as the target nucleotide sequence exists in the target nucleic acid in the sample, signal generation by the detection probe increases earlier, the number of cycles to reach the threshold decreases, and the Ct value measured decreases.

In another aspect, the present invention is directed to a PCR composition for amplifying a target nucleic acid including i) a guide probe capable of hybridizing with a site other than a detection site of the target nucleic acid, ii) a partial primer including a sequence binding to a site other than a target nucleic acid hybridization site of the guide probe and a sequence binding to the target nucleic acid detection site, and iii) a specific primer that pairs with the partial primer to amplify the target nucleic acid.

In the present invention, the sample includes various samples and is preferably a biological sample analyzed using the method of the present invention. More preferably, the sample may be a sample mixed with a virus species or a sample of a subject (e.g., human, mammal, fish, or the like) infected with a virus, and may be a biological sample derived from plants, animals, humans, fungus, bacteria, or virus. When a sample derived from a mammal or human is analyzed, the sample may be derived from a specific tissue or organ. Representative examples of tissues include connective, skin, muscle or nerve tissue. Representative examples of organs include the eye, brain, lungs, liver, spleen, bone marrow, thymus, heart, lymph, blood, bone, cartilage, pancreas, kidney, gallbladder, stomach, small intestine, testicles, ovaries, uterus, rectum, nervous system, and glandular and internal blood vessels. The biological sample to be analyzed includes any cell, tissue, or fluid from a biological source, or any other medium that can be well analyzed by the present invention, and includes samples from food prepared for consumption by humans and/or animals. In addition, the biological samples to be analyzed include bodily fluid samples, which include blood, serum, plasma, lymph, breast milk, urine, feces, ocular fluid, saliva, semen, brain extracts (e.g., brain fragments), spinal fluids, and appendix, spleen, and tonsil tissue extracts, but are not limited thereto.

In another aspect, the present invention is directed to a kit for detecting a target nucleic acid containing the composition.

In the present invention, the kit may optionally include a reagent required for target nucleic acid amplification reaction such as a buffer, DNA polymerase, DNA polymerase cofactor, and deoxyribonucleotide-5-triphosphate (dNTP). Optionally, the kit of the present invention may also include various oligonucleotide molecules, reverse transcriptases, various buffers and reagents, and antibodies that inhibit DNA polymerase activity. In addition, the optimum amount of reagents used in a particular reaction of the kit can be easily determined by those skilled in the art having learned the disclosure herein. Typically, the device of the present invention may be manufactured in a separate package or compartment containing the aforementioned components.

In one embodiment, the kit may include a comparted carrier means accommodating a sample, a container containing a reagent, a container containing the guide probe and the primer, and a container containing a probe for detecting the amplification product.

The carrier means suitably includes at least one container such as a bottle and tube, and each container contains independent components used in the method of the present invention. Here, those skilled in the art can easily dispense the required formulation in the container.

### [Example]

Hereinafter, the present invention will be described in more detail with reference to the following examples. However, it will be obvious to those skilled in the art that the following examples are provided only for illustration of the present invention and should not be construed as limiting the scope of the present invention based on the subject matter of the present invention.

### Example 1. Amplification and detection of BRAF gene with V600E mutation in human genome

### 1.1 Production of primers and probes

It is known that the human BRAF gene encodes a growth signal transduction protein kinase that functions in the body's signal transduction system and certain mutations in the BRAF gene are associated with cancer (Zaman, A. et al., 2019. Cancers 11:1197.). The GTG>GAG nucleotide sequence mutation located at exon 15, codon 600 of the human BRAF gene described above is referred to as a "V600E mutation".

In order to specifically amplify and detect the V600E mutation of the BRAF gene through the novel target nucleic acid amplification method of the present invention, guide probe 1 capable of specifically hybridizing with the region near codon 600 of the BRAF gene, partial primer 1 having a 3' end part capable of specifically hybridizing with codon 600 of the BRAF gene where V600E mutation occurs or a neighboring area thereof, and specific primer 1 capable of hybridizing with a part of the exon 15 nucleotide sequence of the BRAF gene so as to pair with the partial primer 1 and amplify the target nucleic acid were designed. At this time, the partial nucleotide sequence at the C terminal of the guide probe 1 was allowed to hybridize with the partial nucleotide sequence at the 5' end of the partial primer 1. In addition, the detection probe 1 was designed to detect a product amplified through the designed guide probe and primers (PANAGENE Inc., South Korea). Nucleotide sequences of primers and probes are shown in Tables 1 and 2.

**[Table 1]**

| | Sequences of Primers | | |
|---|---|---|---|
| Name | | SEQ ID NO. | Nucleotide sequence (5' end --> 3' end) |
| Partial primer 1 | | SEQ ID NO. 1 | GTCGTCCGTGTTTTGATTTCTCTG |
| Specific primer 1 | | SEQ ID NO. 2 | GCTTGCTCTGATAGGAAAATGAGAT |

**[Table 2]**

| | Sequences of Probes | | |
|---|---|---|---|
| Name | | SEQ ID NO. | Nucleotide sequence (N terminus --> C terminus) |
| Guide probe 1 | | SEQ ID NO. 3 | *[K]-GGACCCACTCCATCG-*[L35 linker] -CACGGACGAC |
| Detection probe 1 | | SEQ ID NO. 4 | [Dabcyl]-GGTGATTTTGGTCTA-[O linker] - [K] - [FAM] |

The partial primer 1 was produced as a single-stranded oligonucleotide with a nucleotide sequence length of 24, the underlined nucleotide sequences were complementary to the part of the nucleotide sequences of guide probe 1, and the italicized nucleotide sequences were complementary to the region in or near the codon 600 of the BRAF gene in which the V600E mutation occurred.

The guide probe 1 was produced as PNA with a nucleotide sequence length of 25, the italicized nucleotide sequences were complementary to the nucleotide sequence near codon 600 of the BRAF gene, and the underlined nucleotide sequences were complementary to the part of the nucleotide sequence of partial primer 1. In addition, [K (lysine)] was attached to the N-terminus of the guide probe 1, and L35 linker from the manufacturer (PANAGENE Inc., South Korea) was interposed between the region of guide probe 1 complementary to the BRAF gene sequence and the region of guide probe 1 complementary to a part of the nucleotide sequence of partial primer 1.

The detection probe 1 was produced as PNA with a nucleotide sequence length of 15, [Dabcyl] as a quencher was attached to the N-terminus thereof, [FAM] as a reporter was attached to the C-terminus thereof, and [O linker] and [K (lysine)] from the manufacturer (PANAGENE Inc., South Korea) were connected between PNA and [FAM]. When the detection probe 1 was hybridized to an amplification product having a complementary nucleotide sequence, the quencher and the reporter were spaced from each other by the farthest distance, and at this time, the signal (fluorescence) from the reporter was maximized. Therefore, the amplification product could be detected by measuring the signal.

It is difficult to hybridize the partial primer 1 with the nucleotide sequence of the target site alone under polymerase chain reaction (PCR) conditions because the length of the nucleotide sequence complementary to the nucleotide sequence of the target site in the BRAF gene is only 10. When the guide probe 1 to be hybridized is present near the target site, a part of the nucleotide sequence of the guide probe 1 is hybridized with a part of the nucleotide sequence of the partial primer 1 and as a result, easy hybridization with the target site is possible.

The partial primer 1 has only 10 nucleotides in length that can be hybridized with the BRAF gene target nucleic acid nucleotide sequence, which is shorter than conventional primers having 18 to 30 nucleotides in a length that can be hybridized with the target nucleic acid. Therefore, when there are one or more mismatched nucleotide sequences in the region where partial primer 1 hybridizes with the target nucleic acid, the effect of the non-complementary nucleotide sequences on hybridization efficiency is greater than that of conventional primers. As a result, it is possible to more efficiently detect small changes in the nucleotide sequence than conventional primers.

### 1.2 Production and confirmation of amplification products

A polymerase chain reaction composition for forming the target amplification product containing 20 pmoles of guide probe 1, 10 pmoles of partial primer 1, 4 pmoles of specific primer 1, and 3 pmoles of detection probe 1 was prepared under the condition in which about 10,000 copies of wild-type BRAF gene target nucleic acids exist, or the condition in which about 10,000 copies of wild-type BRAF gene target nucleic acids are mixed with about 500 V600E-mutated BRAF gene target nucleic acids, or under the condition in which 10,000 copies of wild-type BRAF gene target nucleic acids are mixed with about 500 V600A (codon 600 GTG>GCG)-mutated BRAF gene target nucleic acids, or under the condition in which about 10,000 copies of wild-type BRAF gene target nucleic acids are mixed with about 500 V600D (codon 600 GTG>GAT)-mutated BRAF gene target nucleic acids, or under the condition in which about 10,000 copies of wild-type BRAF gene target nucleic acids are mixed with about 500 V600G (codon 600 GTG>GGG)-mutated BRAF gene target nucleic acids, or under the condition in which about 10,000 copies of wild-type BRAF gene target nucleic acids are mixed with about 500 V600K (codon 600 GTG>AAG)- mutated BRAF gene target nucleic acids, or under the condition in which about 10,000 copies of wild-type BRAF gene target nucleic acids are mixed with about 500 V600M (codon 600 GTG>ATG)-mutated BRAF gene target nucleic acids, or under the condition in which about 10,000 copies of wild-type BRAF gene target nucleic acids are mixed with about 500 V600R (codon 600 GTG>AGG)-mutated BRAF gene target nucleic acids, or under the condition in which about 10,000 copies of wild-type BRAF gene target nucleic acids are mixed with about 500 K601E (codon 601 AAA>GAA)-mutated BRAF gene target nucleic acids.

The polymerase chain reaction composition includes components such as a buffer, deoxyribonucleotide-5-triphosphate (dNTP), potassium chloride (KCl), magnesium chloride (MgCl₂) and a detergent, in addition to the nucleic acid polymerase (DNA polymerase) commonly used in polymerase chain reaction.

Temperature control for the polymerase chain reaction was performed in a general thermal cycler and includes initial denaturation [95°C, 5 minutes], the first stage of 15 cycles including denaturation [95°C, 20 sec], annealing [63°C, 20 sec], and extension [72°C, 20 sec], and the second step of 50 cycles including denaturation [95°C, 20 sec], measurement [50°C, 30 sec] and annealing & extension [60°C, 1 min]. In the measurement of the second step, the fluorescence value of the FAM channel was measured at every cycle to obtain an amplification curve.

As a result, an increase in the fluorescence signal on the amplification curve was observed under all target nucleic acid injection conditions, but the increase in fluorescence signal on the amplification curve was rapid under the condition where the V600E-mutated target nucleic acid occurred, compared to under other conditions, resulting in a lower Ct. In other words, an average Ct of 22.5 was observed under the condition in which the V600E-mutated target nucleic acid was mixed, whereas an average Ct of 29 or more was observed under the condition in which only a wild-type BRAF gene target nucleic acid is present or under the condition in which other target nucleic acids (V600A, V600D, V600G, V600K, V600M, V600R, K601E) are mixed. As a result, only the V600E mutation could be specifically identified through the difference in Ct values (FIG. 5).

### Example 2. High-sensitivity amplification and detection of KRAS gene with G12C mutation in human genome

### 2.1 Production of primers and probes

It is known that the human KRAS gene encodes a GTP-based switch protein that functions in the body's signal transduction system and certain mutations in the KRAS gene are associated with cancer (Waters, A. M. and Der, C. J. 2018. Cold Spring Harb. Perspect. Med. 8:a031435.). The GGT>TGT nucleotide sequence mutation located at exon 2, codon 12 of the human KRAS gene described above is referred to as a "G12C mutation".

In order to specifically amplify and detect the G12C mutation of the KRAS gene through the novel target nucleic acid amplification method of the present invention, guide probe 2 capable of specifically hybridizing with the region near codon 12 of the KRAS gene, partial primer 2 having a 3' end part capable of specifically hybridizing with codon 12 of the KRAS gene where G12C mutation occurs or a neighboring area thereof, and specific primer 2 capable of hybridizing with a part of the exon 2 nucleotide sequence of the KRAS gene so as to pair with the partial primer 2 and amplify the target nucleic acid were designed. At this time, the partial nucleotide sequence at the C terminal of the guide probe 2 was allowed to hybridize with the partial nucleotide sequence at the 5' end of the partial primer 2. In addition, the detection probe 2 was designed to detect a product amplified through the designed guide probe and primers (PANAGENE Inc., South Korea). Nucleotide sequences of primers and probes are shown in Tables 3 and 4.

**[Table 3]**

| | Sequences of primers | | |
|---|---|---|---|
| Name | | SEQ ID NO. | Nucleotide sequence (5' end --> 3' end) |
| Partial primer 2 | | SEQ ID NO. 5 | GCGTGTCCAG*GAGCTTGT* |
| Specific primer 2 | | SEQ ID NO. 6 | TGTATCAAAGAATGGTCCTGCAC |

**[Table 4]**

| | Sequences of probes | | |
|---|---|---|---|
| Name | | SEQ ID NO. | Nucleotide sequence (N terminus --> C terminus) |
| Guide probe 2 | | SEQ ID NO. 7 | [K]-*CTTGTGGTAGTTGG*-[L35 linker] |
| | | -CTGGACACGC | |
| Detection probe 2 | SEQ ID NO. 8 | [Dabcyl]-GTCAAGGCACTC-[O linker] - [K] - [FAM] | |

The partial primer 2 was produced as a single-stranded oligonucleotide with a nucleotide sequence length of 18, the underlined nucleotide sequences are complementary to the part of the nucleotide sequences of guide probe 2, and the italicized nucleotide sequences are complementary to the region in or near the codon 12 of the KRAS gene in which the G12C mutation occurred.

The guide probe 2 was produced as PNA with a nucleotide sequence length of 24, the italicized nucleotide sequences were complementary to the nucleotide sequence near codon 12 of the KRAS gene, and the underlined nucleotide sequences were complementary to the part of the nucleotide sequence of partial primer 2. In addition, [K (lysine)] was attached to the N-terminus of the guide probe 2, and L35 linker from the manufacturer (PANAGENE Inc., South Korea) was interposed between the region of guide probe 2 complementary to the KRAS gene sequence and the region of guide probe 2 complementary to the nucleotide sequence of partial primer 2.

The detection probe 2 was produced as PNA with a nucleotide sequence length of 12, [Dabcyl] as a quencher was attached to the N-terminus thereof, [FAM] as a reporter was attached to the C-terminus thereof, and [O linker] and [K (lysine)] from the manufacturer (PANAGENE Inc., South Korea) were connected between PNA and [FAM]. When the detection probe 2 was hybridized to an amplification product having a complementary nucleotide sequence, the quencher and the reporter were spaced from each other by the farthest distance, and at this time, and the signal (fluorescence) value from the reporter was maximized. Therefore, the amplification product could be detected by measuring the signal.

It is difficult to hybridize the partial primer 2 with the nucleotide sequence of the target site alone under polymerase chain reaction (PCR) conditions because the length of the nucleotide sequence complementary to the nucleotide sequence of the target site in the KRAS gene is only 8. When the guide probe 2 to be hybridized is present near the target site, a part of the nucleotide sequence of the guide probe 2 is hybridized with a part of the nucleotide sequence of the partial primer 2 and as a result, easy hybridization with the target site is possible.

The partial primer 2 has only 8 nucleotides in length that can be hybridized with the KRAS gene target nucleic acid nucleotide sequence, which is shorter than conventional primers having 18 to 30 nucleotides in a length that can be hybridized with the target nucleic acid. Therefore, when there are one or more mismatched nucleotide sequences in the region where partial primer 2 hybridizes with the target nucleic acid, the effect of the non-complementary nucleotide sequences on hybridization efficiency is greater than that of conventional primers. As a result, it is possible to more efficiently detect small changes in the nucleotide sequence than conventional primers.

### 2.2 Production and confirmation of amplification products

A polymerase chain reaction composition for forming the target amplification product containing 20 pmoles of guide probe 2, 10 pmoles of partial primer 2, 4 pmoles of specific primer 2, and 3 pmoles of detection probe 2 was prepared under the condition in which about 10,000 copies of wild-type KRAS gene target nucleic acids exist, or the condition in which about 10,000 copies of wild-type KRAS gene target nucleic acids are mixed with about 100 G12C mutated KRAS gene target nucleic acids, or under the condition in which 10,000 copies of wild-type KRAS gene target nucleic acids are mixed with about 50 G12C mutated KRAS gene target nucleic acids.

The polymerase chain reaction composition includes components such as a buffer, deoxyribonucleotide-5-triphosphate (dNTP), potassium chloride (KCl), magnesium chloride (MgCl₂) and a detergent, in addition to the nucleic acid polymerase (DNA polymerase) commonly used in polymerase chain reaction.

Temperature control for the polymerase chain reaction was performed in a general thermal cycler and includes initial denaturation [95°C, 5 minutes], the first stage of 15 cycles including denaturation [95°C, 20 sec], annealing [63°C, 20 sec], and extension [72°C, 20 sec], and the second step of 35 cycles including denaturation [95°C, 10 sec], measurement [50°C, 10 sec], denaturation [95°C, 20 sec], annealing [58°C, 20 sec], and extension [72°C, 20 sec]. In the measurement of the second step, the fluorescence value of the FAM channel was measured at every cycle to obtain an amplification curve.

As a result, an increase in the fluorescence signal on the amplification curve was clearly observed under all conditions in which the G12C-mutated KRAS target nucleic acid was mixed, whereas there was no or very small increase in the fluorescence signal on the amplification curve under the condition where only the wild-type KRAS target nucleic acid was present. In other words, average Ct values of 12.4 and 13.6 were respectively observed under the condition in which about 100 or about 50 G12C-mutated target nucleic acids were mixed, whereas Ct values were not determined (ND) under the condition where only a wild-type target nucleic acid was present. As a result, the G12C mutation present in very small amounts could be specifically identified through the difference in Ct values (FIG. 6).

### Example 3. Amplification and detection of MET gene with D1002N, D1002G, Y1003F, Y1003N, and Y1003* mutation in human genome

### 3.1 Production of primers and probes

It is known that the human MET gene is also called "c-Met" gene and encodes a hepatocyte growth factor receptor that functions in the body's signal transduction system, and certain mutations and expression dysregulation of the MET gene are associated with cancer (Sierra, J. R. and Tsao, M.-S. 2011. Ther. Adv. Med. Oncol. 3:S2). The GAC>AAC nucleotide sequence mutation located at exon 14, codon 1002 of the human MET gene described above is referred to as a "D1002N mutation", the GAC>GGC nucleotide sequence mutation located at exon 14, codon 1002 of the human MET gene described above is referred to as a "D1002G mutation", the TAC>TTC nucleotide sequence mutation located at exon 14, codon 1003 of the human MET gene described above is referred to as a "Y1003F mutation", the TAC>AAC nucleotide sequence mutation located at exon 14, codon 1003 of the human MET gene described above is referred to as a "Y1003N mutation", and TAC>TAG and TAC>TAA nucleotide sequence mutations located at exon 14, codon 1003 of the human MET gene described above is referred to as a "Y1003*(1)" and "Y1003*(2)", respectively.

In order to specifically amplify and detect the D1002N, D1002G, Y1003F, Y1003N, Y1003*(1), and Y1003*(2) mutations of the MET gene through the novel target nucleic acid amplification method of the present invention, guide probe 3 capable of specifically hybridizing with the region near codon 1002 and codon 1003 of the MET gene, partial primer 3 having a 3' end part capable of specifically hybridizing with the region in or near codon 1002 of the MET gene where D1002N mutation occurs, partial primer 4 having a 3' end part capable of specifically hybridizing with the region in or near codon 1002 of the MET gene where D1002G mutation occurs, partial primer 5 having a 3' end part capable of specifically hybridizing with the region in or near codon 1003 of the MET gene where Y1003F mutation occurs, partial primer 6 having a 3' end part capable of specifically hybridizing with the region in or near codon 1003 of the MET gene where Y1003N mutation occurs, partial primer 7 having a 3' end part capable of specifically hybridizing with the region in or near codon 1003 of the MET gene where Y1003*(1) mutation occurs, partial primer 8 having a 3' end part capable of specifically hybridizing with the region in or near codon 1003 of the MET gene where Y1003*(2) mutation occurs, and specific primer 3 capable of hybridizing with a part of the exon 14 nucleotide sequence of the MET gene so as to pair with the partial primers 3, 4, 5, 6, 7 and 8, and amplify the target nucleic acid were designed. At this time, partial nucleotide sequences at the C terminal of the guide probe 3 were allowed to hybridize with partial nucleotide sequences at the 5' terminals of partial primers 3, 4, 5, 6, 7, and 8. In addition, the detection probe 3 was designed to detect products amplified through the designed guide probe and primers (PANAGENE Inc., South Korea). Nucleotide sequences of primers and probes are shown in Tables 5 and 6.

**[Table 5]**

| | Sequences of primers | | |
|---|---|---|---|
| Name | | SEQ ID NO. | Nucleotide sequence (5' end --> 3' end) |
| Partial primer 3 | | SEQ ID NO. 9 | GTCGTCCGTGTTTA*CGGTAGTTT* |
| Partial primer 4 | | SEQ ID NO. 10 | GTCGTCCGTGTTTT*GTAGCCTA* |
| Partial primer 5 | | SEQ ID NO. 11 | GTCGTCCGTGTTTT*CTCGGAAGT* |
| Partial primer 6 | | SEQ ID NO. 12 | GTCGTCCGTGTTTT*CTCGGTTGT* |
| Partial primer 7 | | SEQ ID NO. 13 | GTCGTCCGTGTTTT*CTCGCTAGT* |
| Partial primer 8 | | SEQ ID NO. 14 | GTCGTCCGTGTTTT*CTCGTTAGT* |
| Specific primer 3 | | SEQ ID NO. 15 | CCATGATAGCCGTCTTTAACA |

**[Table 6]**

| Sequences of Probes | | | |
|---|---|---|---|
| Name | SEQ ID NO. | Nucleotide sequence (N terminus --> C terminus) | |
| Guide probe 3 | SEQ ID NO. 16 | [K]*-CTTCTGGAAAAGTAG-*[L35 linker] -CACGGACGAC | |
| Detection probe 3 | SEQ ID NO. 17 | [Dabcyl]-TACGATGCAAG-[O linker] - [K] - [FAM] | |

The partial primer 3 was produced as a single-stranded oligonucleotide with a nucleotide sequence length of 23, the underlined nucleotide sequences are complementary to the part of the nucleotide sequences of guide probe 3, and the italicized nucleotide sequences are complementary to the region in or near codon 1002 of the MET gene in which the D1002N mutation occurred.

The partial primer 4 was produced as a single-stranded oligonucleotide with a nucleotide sequence length of 22, the underlined nucleotide sequences are complementary to the part of the nucleotide sequences of guide probe 3, and the italicized nucleotide sequences are complementary to the region in or near codon 1002 of the MET gene in which the D1002G mutation occurred.

The partial primer 5 was produced as a single-stranded oligonucleotide with a nucleotide sequence length of 23, the underlined nucleotide sequences are complementary to the part of the nucleotide sequences of guide probe 3, and the italicized nucleotide sequences are complementary to the region in or near codon 1003 of the MET gene in which the Y1003F mutation occurred.

The partial primer 6 was produced as a single-stranded oligonucleotide with a nucleotide sequence length of 23, the underlined nucleotide sequences are complementary to the part of the nucleotide sequences of guide probe 3, and the italicized nucleotide sequences are complementary to the region in or near codon 1003 of the MET gene in which the Y1003N mutation occurred.

The partial primer 7 was produced as a single-stranded oligonucleotide with a nucleotide sequence length of 23, the underlined nucleotide sequences are complementary to the part of the nucleotide sequences of guide probe 3, and the italicized nucleotide sequences are complementary to the region in or near codon 1003 of the MET gene in which the Y1003*(1) mutation occurred.

The partial primer 8 was produced as a single-stranded oligonucleotide with a nucleotide sequence length of 23, the underlined nucleotide sequences are complementary to the part of the nucleotide sequences of guide probe 3, and the italicized nucleotide sequences are complementary to the region in or near codon 1003 of the MET gene in which the Y1003*(2) mutation occurred.

The guide probe 3 was produced as PNA with a nucleotide sequence length of 25, the italicized nucleotide sequences were complementary to the nucleotide sequence near codon 1002 and codon 1003 of the MET gene, and the underlined nucleotide sequences were complementary to the part of the nucleotide sequence of partial primers 3, 4, 5, 6, 7 and 8. In addition, [K (lysine)] was attached to the N-terminus of the guide probe 3, and L35 linker from the manufacturer (PANAGENE Inc., South Korea) was interposed between the region of guide probe 3 complementary to the MET gene sequence and the region of guide probe 3 complementary to a part of the nucleotide sequence of partial primers 3, 4, 5, 6, 7, and 8.

The detection probe 3 was produced as PNA with a nucleotide sequence length of 11, [Dabcyl] as a quencher was attached to the N-terminus thereof, [FAM] as a reporter was attached to the C-terminus thereof, and [O linker] and [K (lysine)] from the manufacturer (PANAGENE Inc., South Korea) were connected between PNA and [FAM]. When the detection probe 3 was hybridized to an amplification product having a complementary nucleotide sequence, the quencher and the reporter were spaced from each other by the farthest distance, and at this time, the signal (fluorescence) from the reporter was maximized. Therefore, the amplification product could be detected by measuring the signal.

It is difficult to hybridize the partial primers 3, 4, 5, 6, 7 and 8 with the nucleotide sequence of the target site alone under polymerase chain reaction (PCR) conditions because the length of the nucleotide sequence complementary to the nucleotide sequence of the target site in the MET gene is only 8 to 9. When the guide probe 3 to be hybridized is present near the target site, a part of the nucleotide sequence of the guide probe 3 is hybridized with a part of the nucleotide sequence of the partial primers 3, 4, 5, 6, 7, and 8, and as a result, easy hybridization with the target site is possible.

The partial primers 3, 4, 5, 6, 7, and 8 have only 8 to 9 nucleotides in length that can be hybridized with the MET gene target nucleic acid nucleotide sequence, which is shorter than conventional primers having 18 to 30 nucleotides in a length that can be hybridized with the target nucleic acid. Therefore, when there are one or more mismatched nucleotide sequences in the region where partial primers 3, 4, 5, 6, 7 and 8 hybridize with the target nucleic acid, the effect of the non-complementary nucleotide sequences on hybridization efficiency is greater than that of conventional primers. As a result, it is possible to more efficiently detect small changes in the nucleotide sequence than conventional primers.

### 3.2 Production and confirmation of amplification products

A polymerase chain reaction composition for forming the target amplification product containing 30 pmoles of guide probe 3, 8 pmoles of partial primer 3, 4 pmoles of partial primer 4, 4 pmoles of partial primer 5, 4 pmoles of partial primer 6, 4 pmoles of partial primer 7, 4 pmoles of partial primer 8, 3 pmoles of specific primer 3, and 2 pmoles of detection probe 3 was prepared under the condition in which about 10,000 copies of wild-type MET gene target nucleic acids exist, or the condition in which about 10,000 copies of wild-type MET gene target nucleic acids are mixed with about 500 D1002N-mutated MET gene target nucleic acids, or under the condition in which 10,000 copies of wild-type MET gene target nucleic acids are mixed with about 500 D1002G-mutated MET gene target nucleic acids, or under the condition in which about 10,000 copies of wild-type MET gene target nucleic acids are mixed with about 500 Y1003F-mutated MET gene target nucleic acids, or under the condition in which about 10,000 copies of wild-type MET gene target nucleic acids are mixed with about 500 Y1003N-mutated MET gene target nucleic acids, or under the condition in which about 10,000 copies of wild-type MET gene target nucleic acids are mixed with about 500 Y1003*(1) mutated MET gene target nucleic acids, or under the condition in which about 10,000 copies of wild-type MET gene target nucleic acids are mixed with about 500 Y1003*(2) mutated MET gene target nucleic acids.

The polymerase chain reaction composition includes component such as a buffer, deoxyribonucleotide-5-triphosphate (dNTP), potassium chloride (KCl), magnesium chloride (MgCl₂) and a detergent, in addition to the nucleic acid polymerase (DNA polymerase) commonly used in polymerase chain reaction.

Temperature control for the polymerase chain reaction was performed in a general thermal cycler and includes initial denaturation [95°C, 5 minutes], the first stage of 15 cycles including denaturation [95°C, 20 sec], annealing [63°C, 20 sec], and extension [72°C, 20 sec], and the second step of 35 cycles including denaturation [95°C, 10 sec], measurement [50°C, 10 sec], denaturation [95°C, 20 sec], annealing [58°C, 20 sec], and extension [72°C, 20 sec]. In the measurement of the second step, the fluorescence value of the FAM channel was measured at every cycle to obtain an amplification curve.

As a result, an increase in the fluorescence signal on the amplification curve was observed under all target nucleic acid injection conditions, but the increase in fluorescence signal on the amplification curve was rapid under the conditions where the D1002N, D1002G, Y1003F, Y1003N, Y1003* (1), and Y1003* (2) mutated target nucleic acids were mixed, compared to the condition in which only wild-type MET gene target nucleic acids were injected, resulting in lower Cts. In other words, an average Ct of 19.9 was observed under the condition in which the D1002N-mutated target nucleic acid was mixed, an average Ct of 18.6 was observed under the condition in which the D1002G-mutated target nucleic acid was mixed, an average Ct of 21.2 was observed under the condition in which the Y1003F-mutated target nucleic acid was mixed, an average Ct of 17.5 was observed under the condition in which the Y1003N-mutated target nucleic acid was mixed, an average Ct of 17.5 was observed under the condition in which the Y1003* (1)-mutated target nucleic acid was mixed, and an average Ct of 20.6 was observed under the condition in which the Y1003* (2)-mutated target nucleic acid was mixed, whereas an average Ct of 28 was observed when only a wild-type nucleic acid was present. As a result, all mutations to be detected could be specifically identified in a single reaction solution through the difference in Ct values (FIG. 7).

### Example 4. Amplification and detection of KRAS gene with G12C mutation in human genome

### 4.1 Production of primers and probes

In order to specifically amplify and detect G12C mutation (GGT>TGT nucleotide sequence mutation) located in exon 2, codon 12 of human KRAS gene through the novel target nucleic acid amplification method, guide probe 4 capable of specifically hybridizing with the region near codon 12 of the KRAS gene, partial primer 2 having a 3' end part capable of specifically hybridizing with the region in or near codon 12 of the KRAS gene, and specific primer 2 capable of hybridizing with a part of the exon 2 nucleotide sequence of the KRAS gene so as to pair with the partial primer 2 and amplify the target nucleic acid were designed. At this time, the partial nucleotide sequence at the C terminal of the guide probe 4 was allowed to hybridize with the partial nucleotide sequence at the 5' end of the partial primer 2. In addition, a detection probe 2 was designed to detect a product amplified through the designed guide probe and primers (PANAGENE Inc., South Korea).

Meanwhile, in order to specifically amplify and detect the KRAS gene in the sample, regardless of the presence or absence of the G12C mutation in the KRAS gene, guide probe 5 capable of specifically hybridizing with the region near exon 6 of the KRAS gene, partial primer 9 having a 3' end part capable of specifically hybridizing with the region in or near exon 6 of the KRAS gene, and specific primer 4 capable of hybridizing with a part of the exon 6 nucleotide sequence of the KRAS gene so as to pair with the partial primer 9 and amplify the target nucleic acid were designed. At this time, the partial nucleotide sequence at the C terminus of the guide probe 5 was allowed to hybridize with the partial nucleotide sequence at the 5' end of the partial primer 9. In addition, a detection probe 4 was designed to detect a product amplified through the designed guide probe and primers (PANAGENE Inc., South Korea). Nucleotide sequences of primers and probes are shown in Tables 7 and 8.

**[Table 7]**

| | Sequences of primers | | |
|---|---|---|---|
| Name | | SEQ ID NO. | Nucleotide sequence (5' end --> 3' end) |
| Partial primer 2 | | SEQ ID NO. 5 | GCGTGTCCAG*GAGCTTGT* |
| Specific primer 2 | | SEQ ID NO. 6 | TGTATCAAAGAATGGTCCTGCAC |
| Partial primer 9 | | SEQ ID NO. 18 | GTCGTCCGTG*CCTTCCACA* |
| Specific primer 4 | | SEQ ID NO. 19 | TGTAGGGCATTTCTGATGTGACTC |

**[Table 8]**

| | Sequences of probes | | |
|---|---|---|---|
| Name | | SEQ ID NO. | Nucleotide sequence (N terminus --> C terminus) |
| Guide probe 4 | | SEQ ID NO. 20 | [K]-*ACTTGTGGTAGTTG*-[L35 linker] -CTGGACACGC |
| Detection probe 2 | | SEQ ID NO. 8 | [Dabcyl]-GTCAAGGCACTC-[O linker] - [K] - [FAM] |
| Guide probe 5 | | SEQ ID NO. 21 | [K]*-AGTGTCATCTTGCCT*-[L35 linker] -CACGGACGAC |
| Detection probe 4 | | SEQ ID NO. 22 | [Dabcyl]-CAGCAGTAAATCT-[O linker] -[K]-[HEX] |

The partial primer 2 was produced as a single-stranded oligonucleotide with a nucleotide sequence length of 18, the underlined nucleotide sequences were complementary to the part of the nucleotide sequences of guide probe 4, and the italicized nucleotide sequences were complementary to the region in or near codon 12 of the KRAS gene in which the G12C mutation occurred.

The partial primer 9 was produced as a single-stranded oligonucleotide with a nucleotide sequence length of 19, the underlined nucleotide sequences were complementary to the part of the nucleotide sequences of guide probe 5, and the italicized nucleotide sequences were complementary to the region in or near exon 6 of the KRAS gene.

The guide probe 4 was produced as PNA with a nucleotide sequence length of 24, the italicized nucleotide sequences were complementary to the nucleotide sequence near codon 12 of the KRAS gene, and the underlined nucleotide sequences were complementary to the part of the nucleotide sequence of partial primer 2. In addition, [K (lysine)] was attached to the N-terminus of the guide probe 4, and L35 linker from the manufacturer (PANAGENE Inc., South Korea) was interposed between the region of guide probe 4 complementary to the KRAS gene sequence and the region of guide probe 4 complementary to a part of the nucleotide sequence of partial primer 2.

The guide probe 5 was produced as PNA with a nucleotide sequence length of 25, the italicized nucleotide sequences were complementary to the nucleotide sequence near exon 6 of the KRAS gene, and the underlined nucleotide sequences were complementary to the part of the nucleotide sequence of partial primer 9. In addition, [K (lysine)] was attached to the N-terminus of the guide probe 5, and L35 linker from the manufacturer (PANAGENE Inc., South Korea) was interposed between the region of guide probe 5 complementary to the KRAS gene sequence and the region of guide probe 5 complementary to a part of the nucleotide sequence of partial primer 9.

The detection probe 2 was produced as PNA with a nucleotide sequence length of 12, [Dabcyl] as a quencher was attached to the N-terminus thereof, [FAM] as a reporter was attached to the C-terminus thereof, and [O linker] and [K (lysine)] from the manufacturer (PANAGENE Inc., South Korea) were connected between PNA and [FAM].

The detection probe 4 was produced as PNA with a nucleotide sequence length of 13, [Dabcyl] as a quencher was attached to the N-terminus thereof, [HEX] as a reporter was attached to the C-terminus thereof, and [O linker] and [K (lysine)] from the manufacturer (PANAGENE Inc., South Korea) were connected between PNA and [HEX].

When the detection probes 2 and 4 were hybridized to amplification products having a complementary nucleotide sequence, the quencher and the reporter were spaced from each other by the farthest distance, and at this time, the signal (fluorescence) from the reporter was maximized. Therefore, the amplification product could be detected by measuring the signal.

It is difficult to hybridize the partial primer 2 with the nucleotide sequence of the target site alone under polymerase chain reaction (PCR) conditions because the length of the nucleotide sequence complementary to the nucleotide sequence of the target site in the KRAS gene is only 8. When the guide probe 4 to be hybridized is present near the target site, a part of the nucleotide sequence of the guide probe 4 is hybridized with a part of the nucleotide sequence of the partial primer 2 and as a result, easy hybridization with the target site is possible.

The partial primer 2 has only 8 nucleotides in length that can be hybridized with the KRAS gene target nucleic acid nucleotide sequence, which is shorter than conventional primers having 18 to 30 nucleotides in a length that can be hybridized with the target nucleic acid. Therefore, when there are one or more mismatched nucleotide sequences in the region where partial primer 2 hybridizes with the target nucleic acid, the effect of the non-complementary nucleotide sequences on hybridization efficiency is greater than that of conventional primers. As a result, it is possible to more efficiently detect small changes in the nucleotide sequence than with conventional primers.

It is difficult to hybridize the partial primer 9 with the nucleotide sequence of the target site alone under polymerase chain reaction (PCR) conditions because the length of the nucleotide sequence complementary to the nucleotide sequence of the target site in the KRAS gene is only 9. When the guide probe 5 to be hybridized is present near the target site, a part of the nucleotide sequence of the guide probe 5 is hybridized with a part of the nucleotide sequence of the partial primer 9 and as a result, easy hybridization with the target site is possible.

The 3' end of the partial primer 9 hybridizes with a part of the exon 6 nucleotide sequence of the KRAS gene, which can occur regardless of the presence or absence of the G12C mutation in the KRAS gene. In consideration of this property, amplification using the partial primer 9 can be used as a control.

That is, unless the G12C mutation of the KRAS gene exists in the sample, amplification through the partial primer 2 (target amplification) is suppressed, while amplification through the partial primer 9 (control amplification) is active, thus resulting in great difference in calculated Ct (ΔCt) between the two amplification curves. On the other hand, when the G12C mutation of the KRAS gene was present in the sample, amplification through both partial primer 2 and partial primer 9 is active, thus resulting in small difference in calculated Ct (ΔCt) between the two amplification curves. Based thereon, the presence of the G12C mutation can be easily determined.

### 4.2 Production and confirmation of amplification products

A polymerase chain reaction composition for forming the target amplification product containing 30 pmoles of guide probe 4, 15 pmoles of guide primer 5, 25 pmoles of partial primer 2, 7 pmoles of partial primer 9, 3 pmoles of specific primer 2, 2 pmoles of specific primer 4, 3 pmoles of detection probe 2, and 3 pmoles of detection probe 4 was prepared under the condition in which about 4,000 copies of wild-type KRAS gene target nucleic acids exist, or the condition in which about wild-type KRAS gene target nucleic acids are mixed with G12C-mutated KRAS gene target nucleic acids at a ratio of 0:100 (100% G12C), 50:50 (50% G12C), 90:10 (10% G12C), 96:4 (4% G12C), 98:2 (2% G12C), 99:1 (1% G12C), or 99.5:0.5 (0.5% G12C) so that the sum of the wild-type KRAS gene target nucleic acids and the G12C mutated KRAS gene target nucleic acids was about 4,000 copies, or under the condition in which wild-type KRAS gene target nucleic acids were mixed with KRAS gene target nucleic acids with non-target mutations, G12A (codon 12 changed to GGT>GCT), G12D (codon 12 changed to GGT>GAT), G12R (codon 12 changed to GGT>CGT), G12S (codon 12 changed to GGT>AGT), G12V (codon 12 changed to GGT>GTT), G13C (codon 13 changed to GGC>TGC), or G13D (codon 13 changed to GGC>GAC) at a ratio of 90:10 (10% G12A, 10% G12D, 10% G12R, 10% G12S, 10% G12V, 10% G13C, 10% G13D) so that the sum of the numbers reached about 4,000 copies.

The polymerase chain reaction composition includes components such as a buffer, deoxyribonucleotide-5-triphosphate (dNTP), potassium chloride (KCl), magnesium chloride (MgCl₂) and a detergent, in addition to the nucleic acid polymerase (DNA polymerase) commonly used in polymerase chain reaction.

Temperature control for the polymerase chain reaction was performed in a general thermal cycler and includes initial denaturation [95°C, 5 min], the first stage of 15 cycles including denaturation [95°C, 20 sec], annealing [63°C, 20 sec], and extension [72°C, 20 sec], and the second step of 35 cycles including denaturation [95°C, 10 sec], measurement [50°C, 10 sec], denaturation [95°C, 20 sec], annealing [58°C, 20 sec], and extension [72°C, 20 sec]. In the measurement of the second step, the fluorescence values of the FAM and HEX channels were measured at every cycle to obtain an amplification curve.

The Ct value is calculated from the amplification curve derived from the FAM channel, which is referred to as "Target region amplification Ct", and the Ct value is calculated from the amplification curve derived from the HEX channel, which is referred to as "Control region amplification Ct". The ΔCt was calculated by subtracting the target site amplification Ct from the control region amplification Ct. The result showed that there was a remarkable difference in ΔCt between the condition injected with the G12C mutation and the condition injected with the wild-type or non-targeted mutation.

That is, the case where ΔCt is -5 or more was determined as "G12C mutation positive", and the case where ΔCt is less than -5 was determined as "G12C mutation negative". G12C mutation positive was determined in all reactions injected with 100% G12C, 50% G12C, 10% G12C, 4% G12C, 2% G12C, and 1% G12C, and G12C mutation positive was determined in 6 out of 8 reactions injected with 0.5% G12C. On the other hand, G12C mutation negative was determined under both the wild-type injection condition and the non-target mutation injection condition. The results showed that specificity as well as high sensitivity at a level that could be detected were achieved even if only about 20 to 40 copies of target mutant nucleic acids per reaction were present (FIG. 9) .

### [Industrial applicability]

The method of amplifying target nucleic acids according to the present invention is advantageously capable of amplifying target nucleic acids present at very low concentrations because the guide probe binds to all hybridizable nucleic acids present in the sample to help partial primers to bind to the target nucleic acid detection site, and of detecting the target nucleic acids at high specificity because there is a difference in amplification rate of nucleic acids other than the target nucleic acids due to the sequence specificity of the partial primer, thus being useful for molecular diagnosis, prenatal diagnosis, early diagnosis, cancer diagnosis, genetic-related diagnosis, diagnosis of genetic traits, diagnosis of infectious bacteria, determination of drug-resistant bacteria, forensic medicine, identification of species of organisms and the like.

Although specific configurations of the present invention have been described in detail, those skilled in the art will appreciate that this description is provided to set forth preferred embodiments for illustrative purposes, and should not be construed as limiting the scope of the present invention. Therefore, the substantial scope of the present invention is defined by the accompanying claims and equivalents thereto.

### [Sequence Listing Free Text]

An electronic file is attached.

## Claims

1. A method of amplifying a target nucleic acid comprising:
(a) mixing a nucleic acid isolated from a sample with i) a guide probe capable of hybridizing with a site other than a detection site of the target nucleic acid;
ii) a partial primer including a sequence binding to a site other than a target nucleic acid hybridization site of the guide probe and a sequence binding to the detection site of the target nucleic acid; and
iii) a specific primer capable of pairing with the partial primer to amplify the target nucleic acid,
and performing polymerase chain reaction (PCR); and
(b) determining whether or not an amplification product is present.

2. The method according to claim 1, wherein a site of the guide probe that hybridizes with the target nucleic acid is present at a 5' end or N-terminus and a site of the guide probe that hybridizes with the partial primer is present at a 3' end or C-terminus.

3. The method according to claim 1, wherein the guide probe further comprises a linker between the site of the guide probe that hybridizes with the target nucleic acid and the site of the guide probe that hybridizes with the partial primer.

4. The method according to claim 3, wherein the linker comprises at least one selected from the group consisting of beta-alanine (β-Ala-OH, C3), aminobutyric acid (C4), aminohexanoic acid (C6), aminolauric acid (C12), acetoacetoxyethyl acrylate (AAEA (O-linker)), aminoethoxyethoxyethoxyacetic acid (2-[2-[2-[2-(amino) ethoxy]ethoxy]ethoxy] acetic acid, AEEEA), AEEEEA, DL15 and L35.

5. The method according to claim 1, wherein the guide probe, the partial primer, and the specific primer comprise oligonucleotide, LNA (locked nucleic acid), PNA (peptide nucleic acid), or a mixture thereof.

6. The method according to claim 5, wherein the guide probe is PNA having a nucleotide sequence length of 10 to 500.

7. The method according to claim 6, wherein the guide probe is PNA having a nucleotide sequence length of 20 to 150.

8. The method according to claim 1, wherein the guide probe is hybridized with an opposite strand to one strand of the target nucleic acid with which the specific primer hybridizes.

9. The method according to claim 1, wherein the partial primer further comprises a spacer as a single strand oligonucleotide with a nucleotide sequence length of 1 to 100 between the sequence binding to the site other than the site of the guide probe hybridizing with the target nucleic acid and the sequence binding to the target nucleic acid detection site.

10. The method according to claim 1, wherein the sequence of the partial primer binding to the target nucleic acid detection site is a sequence of 3 to 15 nucleotides.

11. The method according to claim 1, wherein the amplification product has a length of 50 bp to 1 kbp.

12. The method according to claim 1, wherein the step (b) of determining whether or not the amplification product is present is performed using a nucleic acid-binding dye or probe.

13. The method according to claim 12, wherein the nucleic acid-binding dye is selected from the group consisting of ethidium bromide, SYBRⓡ Green I, SYBRⓡ Gold, EvaGreen, YO-PRO-1, SYTO, BEBO and BEXTO.

14. The method according to claim 12, wherein the probe capable of binding to the amplification product is selected from the group consisting of oligonucleotides, LNAs, PNAs, and mixtures thereof.

15. The method according to claim 14, wherein both ends of the probe capable of binding to the amplification product is connected to a reporter and a quencher.

16. The method according to claim 15, wherein the reporter comprises at least one fluorescent material selected from the group consisting of fluorescein, fluorescein chlorotriazinyl, rhodamine green, rhodamine red, tetramethylrhodamine, FITC, Oregon green, Alexa Fluor, FAM, JOE, ROX, HEX, Texas Red, TET, TRITC, TAMRA, cyanine dyes and thiadicarbocyanine dyes.

17. The method according to claim 15, wherein the quencher comprises at least one selected from the group consisting of Dabcyl, TAMRA, Eclipse, DDQ, QSY, Blackberry Quencher, Black Hole Quencher, Qxl, Iowa Black FQ, Iowa Black RQ, and IRDye QC-1.

18. A PCR composition for amplifying a target nucleic acid comprising:
i) a guide probe capable of hybridizing with a site other than a detection site of the target nucleic acid;
ii) a partial primer including a sequence binding to a site other than a target nucleic acid hybridization site of the guide probe and a sequence binding to the detection site of the target nucleic acid; and
iii) a specific primer that pairs with the partial primer to amplify the target nucleic acid.
